Europäisches Patentamt

European Patent Office

Office européen des brevets

(19)

(11) Veröffentlichungsnummer: **0 004 299**
**B1**

(12) **EUROPÄISCHE PATENTSCHRIFT**

(45) Veröffentlichungstag der Patentschrift: **03.02.82**

(51) Int. Cl.³: **A 61 F  1/00**

(21) Anmeldenummer: **79100615.8**

(22) Anmeldetag: **02.03.79**

(54) Tumor-Gallengangprothese.

(30) Priorität: **17.03.78 DE  2811591**

(43) Veröffentlichungstag der Anmeldung:
**03.10.79 Patentblatt 79/20**

(45) Bekanntmachung des Hinweises auf die Patenterteilung:
**03.02.82 Patentblatt 82/5**

(84) Benannte Vertragsstaaten:
**CH FR GB SE**

(56) Entgegenhaltungen:
**FR - A - 1 500 461**

(73) Patentinhaber: **SIGRI ELEKTROGRAPHIT GMBH
Werner von Siemens-Strasse 18
D-8901 Meitingen (DE)**

(72) Erfinder: **Gistinger, Guido, Dipl.-Ing. Dr. techn.
St. Margareth Strasse 7
D-8901 Biberbach (DE)**
Erfinder: **Wolter, Dietmar, Dr.med.
Ihlendieksweg 30
D-2070 Grosshandsdorf (DE)**

Courier Press, Leamington Spa, England.

## Tumor-Gallengangprothese

Die Erfindung betrifft eine Tumor-Gallengangprothese.

Tumoren im Bereich des Leberhilus, welche von den Gallengängen oder von der Leber ausgehen können, führen im allgemeinen zu einer Verlegung der Gallengänge und zur Aufstauung der Gallenflüssigkeit. Durch den Aufstau der Gallenflüssigkeit kommt es zu einer fortschreitenden Gelbsucht mit schwerer Schädigung der Leber und nachfolgenden schweren Veränderungen am gesamten Organismus. Tumoren im Bereich der Leberpforte und auch in den Gallengängen bedingen eine direkte Verengung des Lumens und durch den von außen auf den Gallengang wirkenden Druck indirekt eine Verlegung der ableitenden Gallenwege. Zur Ableitung der aufgestauten Gallenflüssigkeit werden Operationsverfahren angewendet, bei denen die in einem solchen Zustand erweiterten Gallengänge im Bereich der Leber in eine auf die Leber genähte Darmschlinge abgeleitet werden. Mit dieser Methode kann jedoch häufig ein genügender Abfluß nicht erreicht werden und zum anderen werden insbesondere geschwächte Patienten durch den Eingriff stark belastet.

Noch operationsfähige Tumoren werden dabei entfernt und gegebenenfalls wird eine Verbindung zum Resthauptgallengang mit einer Darmschlinge hergestellt. Häufig ist eine Entfernung des Tumors operationstechnisch jedoch nicht mehr möglich und der verschlossene Gallenabschnitt muß durch eine rohrfömige Prothese überbrückt werden. Die Verwendung von Rohren aus thermoplastischen Kunststoffen und Siliconverbindungen für diesen Zweck haben zu keinem befriedigenden Ergebnis geführt, insbesondere da die Rohre in kurzer Zeit verkrusten. Da die Körperverträglichkeit dieser Materialien ebenfalls nicht ausreicht, wachsen die rohrförmigen Prothesen zudem nur ungenügend in das sie umgebende Gewebe ein.

Ein mit dem körpereigenen Gewebe kompatibler, chemisch und mechanisch beständiger Werkstoff ist Kohlenstoff und Graphit, der mit Erfolg als Implantat verwendet wird. Es ist auch bekannt, die als Glaskohlenstoff oder glasartigen Kohlenstoff bezeichnete Kohlenstofform für Herzklappen, Mittelohrprothesen, Zahnwurzeln und als implantierbare Reizelektrode zu verwenden. Rohrförmige Prothesen, z.B. aus Kohlenstoffgeweben und ebenfalls aus starren Formen des Kohlenstoffs und Graphits sind durch die FR—A 1 500 461 bekannt. Es ist ebenfalls bekannt, die Haftung von Prothesen durch Aufrauhen ihrer äußeren Oberfläche zu verbessern (FR—A 2 105 988, FR—A 2 109 931). Es ist offensichtlich, daß für jede der genannten Prothesen bestimmte Werkstoffe vorzuziehen und zur Nutzung der Werkstoffeigenschaften besondere Ausgestaltungen der Prothese nötig sind, wobei wegen der vielfältigen Anforderungsprofile die Brauchbarkeit eines Werkstoffs, z.B. von Glaskohlenstoff, für eine bestimmte Prothese nach dem heutigen Erkenntnisstand nicht vorhergesagt werden kann.

Der Erfindung liegt nun die Aufgabe zugrunde, die bekannten Tumor-Gallengangprothesen derart zu verbessern, daß eine Verkrustung auch über eine lange Betriebszeit nicht erfolgt und das Einwachsen der Prothese in das umgebende Gewebe begünstigt wird.

Die Aufgabe wird erfindungsgemäß mit einer Prothese aus einem Glaskohlenstoff-Rohr gelöst, dessen äußere Mantelfläche durch Mikroporen 3 aufgerauht und mit ein bis zwei Millimeter tiefen Rillen 2 versehen ist, die sich über die gesamte Mantelfläche erstrecken, und dessen innere Mantelfläche eine feinpolierte Oberfläche aufweist.

Glaskohlenstoff erhält man durch Pyrolyse dreidimensional vernetzter Kunstharze, wie beispielsweise Phenolformaldehyd-oder Furanharzen, bei einer mäßigen Aufheizgeschwindigkeit. Der erhaltene Kohlenstoff ist sehr hart und abriebfest und weist glasartige glatte Oberflächen und Bruchflächen auf. Zur Herstellung der vorteilhaft die Form eines durchgehenden geraden oder gekrümmten Rohrs aufweisenden Prothese füllt man zweckmäßig eine entsprechend ausgebildete Gießform mit einem härtbaren Harz und vernetzt das Harz durch Erhitzen der Form auf eine Temperatur von etwa 120 bis 200°C. Der Harzkörper wird dann durch weiteres Erhitzen auf etwa 800 bis 1200°C in einer inerten oder reduzierenden Atmosphäre carbonisiert. Nach einem anderen Verfahren werden rohrartige Formen durch Schleudern hergestellt.

Die Tiefe der Rillen beträgt zweckmäßig 1 bis 2 mm. Die Herstellung der mikroporösen Oberfläche erfolgt im allgemeinen durch starke Oxidationsmittel, wie oxidierend wirkende Säure, z.B. Salpetersäure, durch elektrochemische Oxidation oder durch Sandstrahlen. Besonders zweckmäßig für die Ausbildung einer aufgerauhten äußeren Oberfläche ist eine Temperung der Prothese in einer oxideerenden Atmosphäre, beispielsweise das Erhitzen in Luft oder in einer anderen Sauerstoff, Wasserdampf oder Kohlendioxid enthaltenden Atmosphäre auf ein Temperatur oberhalb von 400°C. Dabei kommt es zu einem geringfügigen Abbrand an der Oberfläche und zur Ausbildung flacher Ätzgrübchen, deren Tiefe bis etwa 100/um beträgt. Da das die Prothese umgebende Körpergewebe in die Vertiefungen der Prothesenoberfläche einwächst, wird durch die beschriebenen Maßnahmen die Haftung der Prothese wesentlich verbessert und unerwünschte Dislokationen werden mit Sicherheit ausgeschlossen.

Die Beschaffenheit der glasartigen glatten Oberfläche eines Rohres aus Glaskohlenstoff reicht im allgemeinen aux, Verkrustungen auszuschließen. Dieser günstige Effekt wird durch eine Politur der inneren Oberfläche des Rohres verstärkt, wobei beispielsweise Diamantpulver, Kreidepulver oder dergleichen als Poliermittel verwendet wird. Die mittlere Rauhtiefe polierter Glaskohlenstoff-Oberflächen beträgt im allgemeinen weniger als 0,1/um.

Die mit der erfindungsgemäßen Prothese erzielten Vorteile bestehen insbesondere darin, daß verglichen mit den herkömmlichen Methoden die Operationszeit erheblich kürzer ist. Die Prothese verkrustet nicht, so daß erneute Aufstauungen Gallenflüssigkeit vermieden werden und schließlich weist die gewebekompatible Prothese ein vorzügliches Anwachsverhalten auf.

Die Erfindung wird im folgenden anhand einer Zeichnung beispielshaft erläutert.

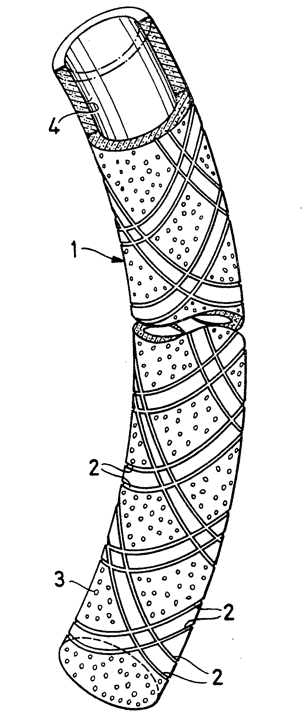

Die in der Figur dargestellte Tumor-Gallengangprothese bosteht aus einem leichtgekrümmten Rohr 1 aus Glaskohlenstoff, dessen äußere Oberfläche mit rillenartigen Vertiefungen 2 versehen und durch flache Mikroporen 3 aufgerauht ist. Die mittlere Tiefe der Rillen beträgt etwa 1,5 mm und die Tiefe der Mikroporen ca. 100/um. Die innere Oberfläche 4 des Rohres 1 ist feinpoliert.

## Patentanspruch

Tumor-Gallengangprothese aus Kohlenstoff, dadurch gekennzeichnet, daß die Prothese aus einem Glaskohlenstoff-Rohr besteht, dessen äußere Mantelfläche durch Mikroporen (3) aufgerauht und mit ein bis zwei Millimeter tiefen Rillen (2) versehen ist, die sich über die gesamte Mantelfläche erstrecken und dessen innere Mantelfläche eine feinpolierte Oberfläche aufweist.

## Claims

A tumor-bile duct prothesis formed of a vitreous carbon tube wherein the outer surface of said tube is roughened by micropores (3) and by grooves (2) having a depth of one to two millimeters and extruding over the whole surface of the tube and wherein the inner surface of said tube is finely-polished.

## Revendication

Vésicule biliaire artificielle utilisée en cas de tumeur, en carbone de verre, caractérisée en ce que la prothèse est constituée par un tube (1) en carbone de verre, dont la surface externe est rendue rugueuse par des micropores (3) et est munie de rainures (2) d'une profondeur de 1 à 2 millimètres, qui s'étendent sur toute la surface externe, et que la surface interne du tube est finement polie.